# EUROPEAN PATENT APPLICATION

(11) **EP 3 903 809 A1**
(43) Date of publication of application: **03.11.2021**
(21) Application number: 20202355.2
(22) Date of filing: 16.10.2020
(51) Int. Cl.: A61K 38/48, A61K 8/34, A61K 8/64, A61K 31/7024

(54) **BOTULINUM TOXIN PHARMACEUTICAL COMPOSITION COMPRISING TANNIC ACID**

(30) Priority: 29.04.2020 KR 20200052074
(71) Applicant: Korea Research Institute of Chemical Technology, Daejeon 34114 (KR)
(72) Inventor: PARK, Sun-Hyun, 34114 Daejeon (KR); KIM, Ki-Suk, 34114 Daejeon (KR); LEE, Jae-Meun, 34114 Daejeon (KR); LEE, Jae-Hyeok, 34114 Daejeon (KR)
(74) Representative: Office Freylinger

(57) **Abstract**

The present invention relates to a botulinum toxin pharmaceutical composition comprising tannic acid as an active ingredient. The botulinum toxin pharmaceutical composition comprising tannic acid provided in one aspect of the present invention improves the activity of the botulinum neurotoxin itself, allows the botulinum neurotoxin to stay in the injected place, and prevents the botulinum neurotoxin from spreading to the blood due to the properties of tannic acid. Therefore, the composition of the present invention has an effect of minimizing the side effects of the conventional Botox caused by the spread of the botulinum neurotoxin.

## Description

### FIELD OF THE INVENTION

The present invention relates to a botulinum toxin pharmaceutical composition comprising tannic acid as an active ingredient.

### BACKGROUND OF THE INVENTION

Anaerobic Clostridium botulinum, grown in canned goods and unsterilized sealed food containers, produces Botulinum toxin (Botox), a potent polypeptide neurotoxin. Neuroparalysis in humans and animals occurs 18 to 36 hours after eating food infected with botulinum toxin or spores. The botulinum toxin structure consists of a heavy chain and a light chain. This toxin passes through the intestinal lining without reducing toxicity, binds to the synaptic vesicle protein exposed during exocytosis of the presynaptic terminal of peripheral motor neurons, and enters the peripheral motor neurons through endocytotic vesicles by clathrin. Due to the high internal acidity of the endocytotic vesicles, the light chain of the botulinum toxin is released into the cytoplasm.

The light chain in the cytoplasm has a zinc endopeptidase function, so it attacks vesicle proteins. As a result, the release of acetylcholine, a neurotransmitter, is suppressed, leading to symptoms of botulinum toxin poisoning, such as gait disturbance, dysphagia, speech disorder, respiration muscle paralysis, etc., and may lead to death.

The molecular weight of the botulinum toxin protein is about 150 kDa, and there are seven botulinum toxins known to date (A, B, C, D, E, F and G). The botulinum toxin is released by Clostridium bacteria as a complex containing a 150 kDa botulinum toxin protein molecule along with a non-toxic protein. Therefore, the botulinum toxin type A complex can be produced by Clostridium bacteria as the forms of 900, 500 and 300 kDa. The botulinum toxin types B and C are produced as a 500 kDa complex, and the botulinum toxin type D is produced as 300 kD and 500 kDa complexes. The botulinum toxin types E and F are produced only as a 300 kD complex. The composition of the complexes is believed to include a non-toxic and non-hemagglutinin protein structure and a hemagglutinin protein. The complex composition includes a non-toxic non-hemagglutinin protein structure and an erythrocyte agglutinin protein. These two non-toxic proteins act to provide stability against botulinum toxin molecule denaturation and protection against digestive acids when the toxin is ingested. It is known that the larger the botulinum toxin complex, the more slowly the botulinum toxin spreads from the intramuscular injection site. When the toxin complex is treated with erythrocytes at pH 7.3, the complex can be separated into a toxin protein and a hemagglutinin protein. When the hemagglutinin protein is separated, the toxin protein becomes significantly unstable.

As commercially available botulinum toxins, BOTOX® (Allergan, Inc., 900 kDa full accessory protein complex), Jeuveau (900 kDa full accessory protein complex), Dysport (Undisclosed complex), Xeomin (150 kDa no accessory proteins), Medytox (Korea), Botulax (Hugel), etc. are available. The composition of most BOTOX®, Jeuveau, Dysport, Medytox and Botulax consists of the purified botulinum toxin type A complex, albumin and sodium chloride, which packaged in sterile, vacuum-dried form. The botulinum toxin type A is prepared from a culture of the Clostridium botulinum Hall strain cultured in a medium containing N-Z amine and yeast extract. The botulinum toxin type A complex is purified from a culture solution through a series of acid precipitation into a crystalline complex consisting of an active high molecular weight toxin protein and a related hemagglutinin protein. The crystalline complex is re-dissolved in brine and albumin, filter sterilized (0.2 micron), and vacuum dried.

Botulinum toxin is used in clinical settings for the treatment of neuromuscular disorders characterized by spasticity (hyperactive skeletal muscle) caused by cerebral palsy and stroke. The botulinum toxin type A was approved by the US Food and Drug Administration in 1989 for the treatment of essential blepharospasm, strabismus and hemifacial spasm in patients 12 years of age or older. The clinical effect of the botulinum toxin type A injected into the peripheral muscle usually occurs within 1 week after the injection. The typical duration of symptom relief (ie, relaxed muscle paralysis) by a single intramuscular injection of botulinum toxin type A is about 3 months. Recently, it has been widely used as a wrinkle improvement treatment to improve women's wrinkles.

Meanwhile, after using a botulinum toxin product for medical or cosmetic purposes, there is a serious problem in that unintended side effects occur. Specifically, the toxin can spread from the injection site to other parts of the body, and cause muscle paralysis. In addition, it may be difficult to breathe and swallow food due to the toxin. More than 600 unwanted side effects and 28 deaths have been reported worldwide due to the toxin. In February 2008, the US Food and Drug Administration (FDA) reported a more serious side effect of the toxin, which is that several children with limb spasm died after treatment. Recently, from pharmaceutical companies, patients, and cosmetic users, there is a growing demand for products that can increase the efficacy of Botox but minimize side effects caused by unintended spread of Botox *in vivo.*

Patent reference 1 (Korean Patent No. 10-2088104) is an example of using Erigeron annuus flower essential oil as a Botox adjuvant for the purpose of increasing the medicinal effect of Botox. More specifically, the patent reference 1 aims to extend the duration of maintenance of the botulinum neurotoxin effect by further including Erigeron annuus flower essential oil in the botulinum neurotoxin composition, and this was confirmed through the maintenance period of muscle paralysis. However, the patent reference 1 does not disclose the necessity of reducing side effects caused by diffusion *in vivo* without staying at the injection site of Botox mentioned above and solutions for minimizing the side effects at all.

The present inventors have conducted studies to derive an adjuvant that can not only improve the activity of Botox itself, but also minimize the side effects that occur when Botox is diffused from the injection site, and a composition containing the same.

While performing the above studies, the present inventors have found that the botulinum toxin pharmaceutical composition comprising tannic acid provided in one aspect of the present invention improves the activity of the botulinum neurotoxin itself, allows the botulinum neurotoxin to stay in the injected place, and prevents the botulinum neurotoxin from spreading to the blood due to the properties of tannic acid. As a result, the present inventors have completed the present invention by confirming the effect of minimizing the side effects of the conventional Botox caused by the spread of the botulinum neurotoxin.

### SUMMARY OF THE INVENTION

It is an object of the present invention to provide a tannylated botulinum toxin complex that can minimize side effects of the conventional Botox by improving the activity of the botulinum neurotoxin itself, and preventing the botulinum neurotoxin from staying in the injected place and spreading to the blood by the properties of tannic acid.

It is another object of the present invention to provide a pharmaceutical composition comprising Botulinum toxin (Botox); and tannic acid (TA) as active ingredients.

It is another object of the present invention to provide a method for treating neuromuscular related diseases comprising a step of administering a pharmaceutically effective amount of a tannylated botulinum toxin complex containing botulinum toxin (Botox) and tannic acid (TA) to a subject in need.

It is another object of the present invention to provide a pharmaceutical composition comprising botulinum toxin (Botox); and tannic acid (TA) as active ingredients, for use in preventing or treating neuromuscular related diseases.

It is another object of the present invention to provide a use of the tannylated botulinum toxin complex containing botulinum toxin (Botox) and tannic acid (TA) to prepare a medicament for the treatment of neuromuscular related diseases.

It is another object of the present invention to provide a cosmetic composition comprising Botulinum toxin (Botox); and tannic acid (TA) as active ingredients.

It is another object of the present invention to provide a method for treating cosmetic defect comprising a step of administering a (cosmetically) effective amount of a tannylated botulinum toxin complex containing botulinum toxin (Botox) and tannic acid (TA) to a subject in need.

It is another object of the present invention to provide a use of the tannylated botulinum toxin complex containing botulinum toxin (Botox) and tannic acid (TA) to prepare a cosmetic composition for the treatment of cosmetic defect.

It is another object of the present invention to provide a botulinum toxin adjuvant comprising tannic acid (TA) as an active ingredient, for use in the prevention or treatment of one or more diseases selected from the group consisting of myoclonus, hemifacial spasm, spasmodic torticollis, anal fissure, blepharospasm, facial spasm, cerebral palsy, medication overuse headache, chronic migraine, myalgia, strabismus, temporomandibular disorder, neuralgia, overactive bladder, urge urinary incontinence, rhinitis, acne, dystonia, hyperhidrosis and vocal fold granuloma.

It is another object of the present invention to provide a botulinum toxin (Botox) adjuvant comprising tannic acid (TA) as an active ingredient.

To achieve the above objects, in an aspect of the present invention, the present invention provides a tannylated botulinum toxin complex comprising botulinum toxin (Botox) and tannic acid (TA).

In another aspect of the present invention, the present invention provides a pharmaceutical composition comprising botulinum toxin (Botox); and tannic acid (TA) as active ingredients.

In another aspect of the present invention, the present invention provides a method for treating neuromuscular related diseases comprising a step of administering a pharmaceutically effective amount of a tannylated botulinum toxin complex containing botulinum toxin (Botox) and tannic acid (TA) to a subject in need.

In another aspect of the present invention, the present invention provides a use of the tannylated botulinum toxin complex containing botulinum toxin (Botox) and tannic acid (TA) to prepare a medicament for the treatment of neuromuscular related diseases.

In another aspect of the present invention, the present invention provides a cosmetic composition comprising botulinum toxin (Botox); and tannic acid (TA) as active ingredients.

In another aspect of the present invention, the present invention provides a method for treating cosmetic defect comprising a step of administering a pharmaceutically effective amount of a tannylated botulinum toxin complex containing botulinum toxin (Botox) and tannic acid (TA) to a subject in need.

In another aspect of the present invention, the present invention provides a use of the tannylated botulinum toxin complex containing botulinum toxin (Botox) and tannic acid (TA) to prepare a medicament for the treatment of cosmetic defect.

In another aspect of the present invention, the present invention provides a botulinum toxin (Botox) adjuvant comprising tannic acid (TA) as an active ingredient.

### ADVANTAGEOUS EFFECT

The botulinum toxin pharmaceutical composition comprising tannic acid provided in one aspect of the present invention improves the activity of the botulinum neurotoxin itself, allows the botulinum neurotoxin to stay in the injected place, and prevents the botulinum neurotoxin from spreading to the blood due to the properties of tannic acid. Therefore, the composition of the present invention has an effect of minimizing the side effects of the conventional Botox caused by the spread of the botulinum neurotoxin.

### BRIEF DESCRIPTION OF THE DRAWINGS

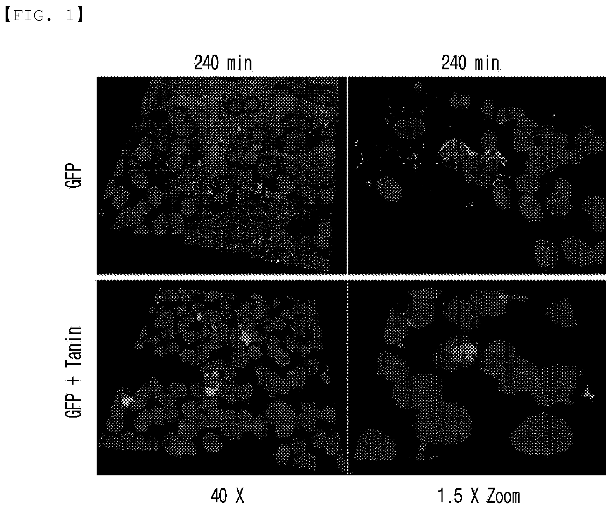

Figure 1 is a set of photographs showing the results of comparing and evaluating the degree of endocytosis of a green fluorescent protein into cells when CHO (Chinese Hamster Ovary) cells are treated with a green fluorescent protein (GFP) alone or a tannylated fluorescent protein prepared by mixing GFP and tannic acid.

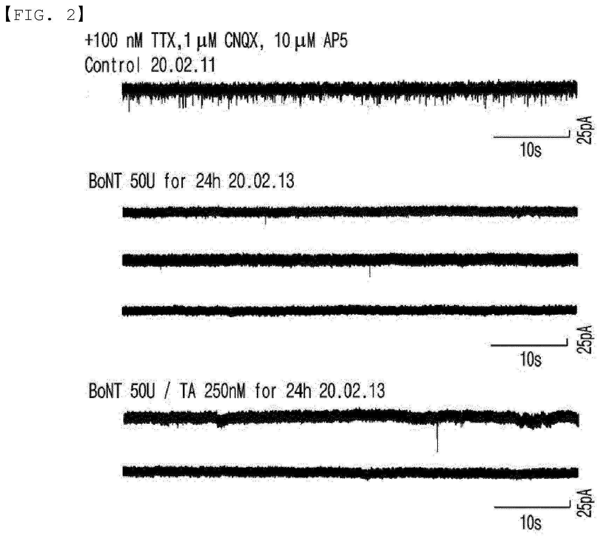

Figure 2 is a diagram showing the results of patch-clamp of the group treated with botulinum toxin (BTX) alone (corresponding to BoNT 50U in the diagram) and the group treated with botulinum toxin (BTX) tannylated by treating tannic acid (TA) (corresponding to BoNT 50U / TA 250 nM in the diagram).

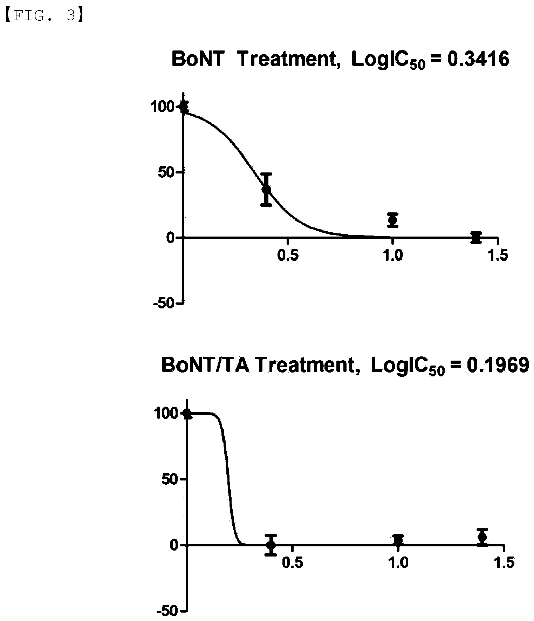

Figure 3 is a set of graphs showing the results of deriving LogIC₅₀ values for each treatment concentration of botulinum toxin from the results of patch-clamp of the group treated with botulinum toxin (BTX) alone (corresponding to BoNT Treatment in the diagram) and the group treated with botulinum toxin (BTX) tannylated by treating tannic acid (TA) (corresponding to BoNT/TA Treatment in the diagram).

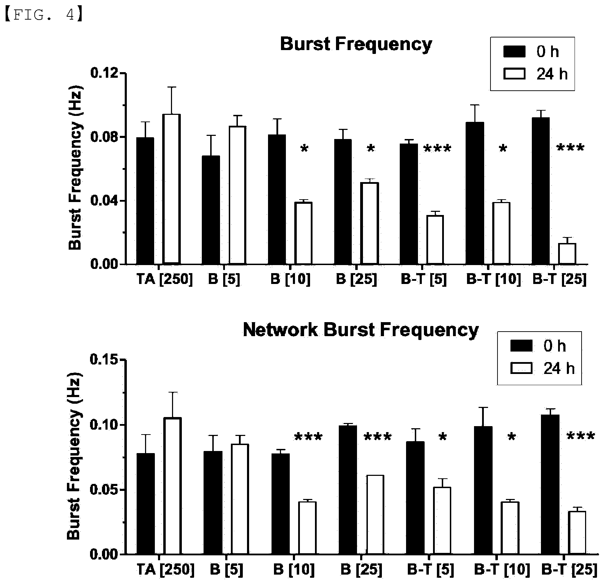

Figure 4 is a set of graphs showing the results of recording burst frequencies and network burst frequencies of the group treated with tannic acid (TA) alone (corresponding to TA[250] in the diagram), the group treated with botulinum toxin (BTX) alone (corresponding to B[5], B[10] and B[25] in the diagram) and the group treated with botulinum toxin (BTX) tannylated by treating tannic acid (TA) (corresponding to B-T[5], B-T[10] and B-T[25] in the diagram) from Axion MEA data. In the case of TA, the concentration unit is nM, in the case of B, the concentration unit is U (unit), the value in [] of B-T is the concentration of B, and the TA concentration is fixed at 250 nM.

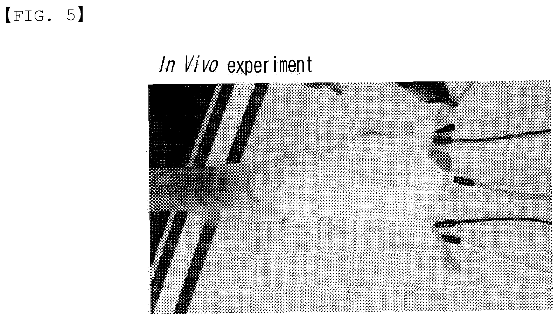

Figure 5 is a diagram showing the experimental process of an *in vivo* experiment evaluating whether the side effects caused by intradermally injecting botulinum toxin can be reduced when botulinum toxin is tannylated by mixing botulinum toxin (BTX) and tannic acid.

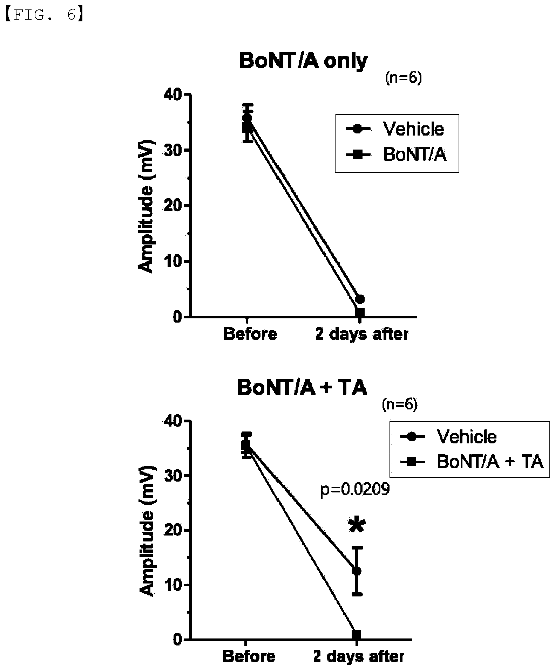

Figure 6 is a set of graphs showing the results of comparing the possibility of causing side effects by expanding botulinum toxin through blood vessels from the injection site by measuring CMAP electrical waveforms at the injection site and the non-injection site (Vehicle) in the group injected with botulinum toxin (BTX) alone (corresponding to BoNT/A only in the diagram) and the group injected with a mixture of botulinum toxin and tannic acid (corresponding to BoNT/A + TA in the diagram).

### DESCRIPTION OF THE PREFERRED EMBODIMENTS

Hereinafter, the present invention is described in detail.

The embodiments of this invention can be modified in various other forms, and the scope of the present invention is not limited to the embodiments described below. It is well understood by those in the art who has the average knowledge on this field that the embodiments of the present invention are given to explain the present invention more precisely.

In addition, the "inclusion" of an element throughout the specification does not exclude other elements, but may include other elements, unless specifically stated otherwise.

In an aspect of the present invention, the present invention provides a tannylated botulinum toxin complex comprising botulinum toxin (Botox) and tannic acid (TA). At this time, the tannic acid is characterized by being tannylated by binding to the botulinum toxin.

In another aspect of the present invention, the present invention provides a pharmaceutical composition comprising botulinum toxin (Botox); and tannic acid (TA) as active ingredients. At this time, the tannic acid is characterized by being tannylated by binding to the botulinum toxin.

The pharmaceutical composition can be used for preventing or treating neuromuscular related diseases.

The molecular weight of the botulinum toxin protein is about 150 kDa, and there are seven botulinum toxins known to date (A, B, C, D, E, F and G). The botulinum toxin is released by Clostridium bacteria as a complex containing a 150 kDa botulinum toxin protein molecule along with a non-toxic protein. Therefore, the botulinum toxin type A complex can be produced by Clostridium bacteria as the forms of 900, 500 and 300 kDa. The botulinum toxin types B and C are produced as a 500 kDa complex, and the botulinum toxin type D is produced as 300 kD and 500 kDa complexes. The botulinum toxin types E and F are produced only as a 300 kD complex. The composition of the complexes includes a non-toxic and non-hemagglutinin protein structure and a hemagglutinin protein. In the present invention, the botulinum toxin (Botox) can be a botulinum neurotoxin type A (BoNT/A).

The botulinum toxin can be included in the composition in a concentration range of 5 to 10,000 U/ml, but is not particularly limited to the concentration range, and the concentration range can be appropriately adjusted according to the purpose of use of the composition.

In one embodiment, several concentration ranges may be, for example, a concentration range of 5 to 8,000 U/ml, a concentration range of 5 to 6,000 U/ml, a concentration range of 5 to 4,000 U/ml, a concentration range of 5 to 2,000 U/ml, a concentration range of 5 to 1,000 U/ml, a concentration range of 5 to 900 U/ml, a concentration range of 5 to 800 U/ml, a concentration range of 5 to 700 U/ml, a concentration range of 5 to 600 U/ml, a concentration range of 5 to 500 U/ml, a concentration range of 5 to 400 U/ml, a concentration range of 5 to 300 U/ml, a concentration range of 5 to 200 U/ml, a concentration range of 5 to 100 U/ml, a concentration range of 5 to 90 U/ml, a concentration range of 5 to 80 U/ml, a concentration range of 5 to 70 U/ml, a concentration range of 5 to 60 U/ml, and a concentration range of 5 to 50 U/ml.

Tannic acid (TA) is one of the most abundant polyphenols found in plants such as fruits, vegetables, olives, and cacao. Recently, tannic acid is used as a multifunctional coating molecule. In addition, TA is known as a molecule having excellent affinity with biomacromolecules including DNA and proline-rich proteins such as thrombin, gelatin, collagen and mucin. Tannic acid can bind to proteins through multiple hydrogen bonds and hydrophobic interactions between the phenolic hydroxyl group rich moiety (5 gallol groups (3 -OH groups linked to an aromatic ring) and 5 catechol groups (2 -OH groups covalently linked to an aromatic ring)) and the target protein.

The tannic acid (TA) can be used in an appropriate amount depending on the amount of botulinum toxin in the composition and/or maximizing the activity of the botulinum toxin itself or minimizing side effects. In one embodiment, the tannic acid can be included in the composition at a concentration range of 10 nM to 500 µM. In another aspect, the tannic acid can be included in the composition at a concentration range of 10 nM to 480 µM, at a concentration range of 10 nM to 460 µM, at a concentration range of 10 nM to 440 µM, at a concentration range of 10 nM to 420 µM, at a concentration range of 10 nM to 400 µM, at a concentration range of 10 nM to 380 µM, at a concentration range of 10 nM to 360 µM, at a concentration range of 10 nM to 340 µM, at a concentration range of 10 nM to 320 µM, at a concentration range of 10 nM to 460 µM, at a concentration range of 10 nM to 300 µM, at a concentration range of 10 nM to 280 µM, at a concentration range of 10 nM to 260 µM, at a concentration range of 10 nM to 240 µM, at a concentration range of 10 nM to 220 µM, at a concentration range of 10 nM to 200 µM, at a concentration range of 10 nM to 180 µM, at a concentration range of 10 nM to 160 µM, at a concentration range of 10 nM to 140 µM, at a concentration range of 10 nM to 120 µM, at a concentration range of 10 nM to 100 µM, at a concentration range of 30 nM to 500 µM, at a concentration range of 50 nM to 500 µM, at a concentration range of 70 nM to 500 µM, at a concentration range of 90 nM to 500 µM, at a concentration range of 110 nM to 500 µM, at a concentration range of 130 nM to 500 µM, at a concentration range of 150 nM to 500 µM, at a concentration range of 170 nM to 500 µM, at a concentration range of 190 nM to 500 µM, at a concentration range of 210 nM to 500 µM, and a concentration range of 230 nM to 500 µM, but not always limited thereto.

The neuromuscular related disease can be selected from the group consisting of myoclonus, hemifacial spasm, spasmodic torticollis, anal fissure, blepharospasm, facial spasm, cerebral palsy, medication overuse headache, chronic migraine, myalgia, strabismus, temporomandibular disorder, neuralgia, overactive bladder, urge urinary incontinence, rhinitis, acne, dystonia, hyperhidrosis and vocal fold granuloma.

The tannic acid (TA) improves the activity of botulinum toxin (Botox) by 1.5 to 10 times and prevents the botulinum toxin from spreading from the injection site, thereby reducing side effects caused by the spread of the botulinum toxin.

In another aspect of the present invention, the present invention provides a pharmaceutical kit for preventing or treating neuromuscular related diseases comprising a first component containing botulinum toxin (Botox); and a second component containing tannic acid (TA).

The botulinum toxin can be included in the first component in a concentration range of 5 to 10,000 U/ml, but is not particularly limited to the concentration range, and the concentration range can be appropriately adjusted according to the purpose of use of the kit. In one embodiment, several concentration ranges may be, for example, a concentration range of 5 to 8,000 U/ml, a concentration range of 5 to 6,000 U/ml, a concentration range of 5 to 4,000 U/ml, a concentration range of 5 to 2,000 U/ml, a concentration range of 5 to 1,000 U/ml, a concentration range of 5 to 900 U/ml, a concentration range of 5 to 800 U/ml, a concentration range of 5 to 700 U/ml, a concentration range of 5 to 600 U/ml, a concentration range of 5 to 500 U/ml, a concentration range of 5 to 400 U/ml, a concentration range of 5 to 300 U/ml, a concentration range of 5 to 200 U/ml, a concentration range of 5 to 100 U/ml, a concentration range of 5 to 90 U/ml, a concentration range of 5 to 80 U/ml, a concentration range of 5 to 70 U/ml, a concentration range of 5 to 60 U/ml, and a concentration range of 5 to 50 U/ml.

In the second component, the tannic acid (TA) can be used in an appropriate amount depending on the amount of botulinum toxin in the kit and/or maximizing the activity of the botulinum toxin itself or minimizing side effects. In one embodiment, the tannic acid can be included in the second component at a concentration range of 10 nM to 500 µM. In another aspect, the tannic acid can be included in the component at a concentration range of 10 nM to 480 µM, at a concentration range of 10 nM to 460 µM, at a concentration range of 10 nM to 440 µM, at a concentration range of 10 nM to 420 µM, at a concentration range of 10 nM to 400 µM, at a concentration range of 10 nM to 380 µM, at a concentration range of 10 nM to 360 µM, at a concentration range of 10 nM to 340 µM, at a concentration range of 10 nM to 320 µM, at a concentration range of 10 nM to 460 µM, at a concentration range of 10 nM to 300 µM, at a concentration range of 10 nM to 280 µM, at a concentration range of 10 nM to 260 µM, at a concentration range of 10 nM to 240 µM, at a concentration range of 10 nM to 220 µM, at a concentration range of 10 nM to 200 µM, at a concentration range of 10 nM to 180 µM, at a concentration range of 10 nM to 160 µM, at a concentration range of 10 nM to 140 µM, at a concentration range of 10 nM to 120 µM, at a concentration range of 10 nM to 100 µM, at a concentration range of 30 nM to 500 µM, at a concentration range of 50 nM to 500 µM, at a concentration range of 70 nM to 500 µM, at a concentration range of 90 nM to 500 µM, at a concentration range of 110 nM to 500 µM, at a concentration range of 130 nM to 500 µM, at a concentration range of 150 nM to 500 µM, at a concentration range of 170 nM to 500 µM, at a concentration range of 190 nM to 500 µM, at a concentration range of 210 nM to 500 µM, and a concentration range of 230 nM to 500 µM, but not always limited thereto.

In another aspect of the present invention, the present invention provides a method for improving the activity of botulinum toxin, comprising a step of tannylating the botulinum toxin by treating the botulinum toxin (Botox) with tannic acid (TA).

In another aspect of the present invention, the present invention provides a method for reducing the side effects caused by *in vivo* diffusion of botulinum toxin, comprising a step of tannylating the botulinum toxin by treating the botulinum toxin (Botox) with tannic acid (TA).

In another aspect of the present invention, the present invention provides a method for treating neuromuscular related diseases comprising a step of administering a pharmaceutically effective amount of a tannylated botulinum toxin complex containing botulinum toxin (Botox) and tannic acid (TA) to a subject in need. At this time, the administration is not particularly limited to oral/parenteral administration, but administration by injection is preferred.

In another aspect of the present invention, the present invention provides a use of the tannylated botulinum toxin complex containing botulinum toxin (Botox) and tannic acid (TA) to prepare a medicament for the treatment of neuromuscular related diseases.

In another aspect of the present invention, the present invention provides a cosmetic composition comprising botulinum toxin (Botox); and tannic acid (TA) .

At this time, the cosmetic effect of the cosmetic composition may be one or more selected from the group consisting of reduced appearance of fine lines, reduced appearance of wrinkles, widening of the eyes, lifting the corner of the mouth, decrease in muscle mass and flattening of the lines extending from the upper lip.

The botulinum toxin can be included in the composition in a concentration range of 5 to 10,000 U/ml, but is not particularly limited to the concentration range, and the concentration range can be appropriately adjusted according to the purpose of use of the cosmetic composition. In one embodiment, several concentration ranges may be, for example, a concentration range of 5 to 8,000 U/ml, a concentration range of 5 to 6,000 U/ml, a concentration range of 5 to 4,000 U/ml, a concentration range of 5 to 2,000 U/ml, a concentration range of 5 to 1,000 U/ml, a concentration range of 5 to 900 U/ml, a concentration range of 5 to 800 U/ml, a concentration range of 5 to 700 U/ml, a concentration range of 5 to 600 U/ml, a concentration range of 5 to 500 U/ml, a concentration range of 5 to 400 U/ml, a concentration range of 5 to 300 U/ml, a concentration range of 5 to 200 U/ml, a concentration range of 5 to 100 U/ml, a concentration range of 5 to 90 U/ml, a concentration range of 5 to 80 U/ml, a concentration range of 5 to 70 U/ml, a concentration range of 5 to 60 U/ml, and a concentration range of 5 to 50 U/ml.

The tannic acid (TA) can be used in an appropriate amount depending on the amount of botulinum toxin in the composition and/or maximizing the activity of the botulinum toxin itself or minimizing side effects. In one embodiment, the tannic acid can be included in the composition at a concentration range of 10 nM to 500 µM. In another aspect, the tannic acid can be included in the composition at a concentration range of 10 nM to 480 µM, at a concentration range of 10 nM to 460 µM, at a concentration range of 10 nM to 440 µM, at a concentration range of 10 nM to 420 µM, at a concentration range of 10 nM to 400 µM, at a concentration range of 10 nM to 380 µM, at a concentration range of 10 nM to 360 µM, at a concentration range of 10 nM to 340 µM, at a concentration range of 10 nM to 320 µM, at a concentration range of 10 nM to 460 µM, at a concentration range of 10 nM to 300 µM, at a concentration range of 10 nM to 280 µM, at a concentration range of 10 nM to 260 µM, at a concentration range of 10 nM to 240 µM, at a concentration range of 10 nM to 220 µM, at a concentration range of 10 nM to 200 µM, at a concentration range of 10 nM to 180 µM, at a concentration range of 10 nM to 160 µM, at a concentration range of 10 nM to 140 µM, at a concentration range of 10 nM to 120 µM, at a concentration range of 10 nM to 100 µM, at a concentration range of 30 nM to 500 µM, at a concentration range of 50 nM to 500 µM, at a concentration range of 70 nM to 500 µM, at a concentration range of 90 nM to 500 µM, at a concentration range of 110 nM to 500 µM, at a concentration range of 130 nM to 500 µM, at a concentration range of 150 nM to 500 µM, at a concentration range of 170 nM to 500 µM, at a concentration range of 190 nM to 500 µM, at a concentration range of 210 nM to 500 µM, and a concentration range of 230 nM to 500 µM, but not always limited thereto.

In another aspect of the present invention, the present invention provides a method for treating cosmetic defect comprising a step of administering a pharmaceutically effective amount of a tannylated botulinum toxin complex containing botulinum toxin (Botox) and tannic acid (TA) to a subject in need. At this time, the administration is not particularly limited to oral/parenteral administration, but administration by injection is preferred. In addition, the cosmetic defect can be wrinkle.

In another aspect of the present invention, the present invention provides a use of the tannylated botulinum toxin complex containing botulinum toxin (Botox) and tannic acid (TA) to prepare a medicament for the treatment of cosmetic defect.

In another aspect of the present invention, the present invention provides a botulinum toxin (Botox) adjuvant comprising tannic acid (TA) as an active ingredient.

The botulinum toxin (Botox) adjuvant can be used for the prevention or treatment of neuromuscular related diseases selected from the group consisting of myoclonus, hemifacial spasm, spasmodic torticollis, anal fissure, blepharospasm, facial spasm, cerebral palsy, medication overuse headache, chronic migraine, myalgia, strabismus, temporomandibular disorder, neuralgia, overactive bladder, urge urinary incontinence, rhinitis, acne, dystonia, hyperhidrosis and vocal fold granuloma.

In addition, the botulinum toxin (Botox) adjuvant can be used for cosmetic purposes. The botulinum toxin (Botox) adjuvant can be administered simultaneously with botulinum toxin.

The botulinum toxin pharmaceutical composition comprising tannic acid provided in one aspect of the present invention improves the activity of the botulinum neurotoxin itself, allows the botulinum neurotoxin to stay in the injected place, and prevents the botulinum neurotoxin from spreading to the blood due to the properties of tannic acid. Therefore, the composition of the present invention has an effect of minimizing the side effects of the conventional Botox caused by the spread of the botulinum neurotoxin. In particular, the pharmaceutical composition according to the present invention comprising botulinum toxin as an active ingredient can minimize side effects by reducing the spread of the botulinum toxin from the sites of high efficiency targeted treatment and intramuscular injection. This is directly supported by the experimental examples described below.

Hereinafter, the present invention will be described in detail by the following examples and experimental examples.

However, the following examples and experimental examples are only for illustrating the present invention, and the contents of the present invention are not limited thereto.

### Experimental Example 1: Evaluation of tannylated protein endocytosis through tannic acid (TA) and protein binding

It was confirmed that tannic acid could deliver proteins into cells by taking advantage of its high binding power to proteins. Because proteins are hydrophilic, they must have receptors or channels for the protein to be delivered into cells. Since tannic acid can bind to all proteins, free OH groups remaining after the binding of tannic acid to proteins can also bind to various proteins in the cell membrane. Cells continue to undergo repetitive endocytosis and exocytosis. If a protein bound to tannic acid binds to the cell membrane, it can be introduced into the cell through endocytosis. The present inventors confirmed whether a mixture of tannic acid and protein was introduced into cells. Particularly, the difference of the degree of endocytosis of a green fluorescent protein into cells in the group wherein CHO (Chinese Hamster Ovary) cells were treated with a tannylated protein and treated with a green fluorescent protein (GFP) after 4 hours, and the group treated with a green fluorescent protein (GFP) conjugated with tannic acid was compared.

The results are shown in Figure 1.

Figure 1 is a set of photographs showing the results of comparing and evaluating the degree of endocytosis of a green fluorescent protein into cells when CHO (Chinese Hamster Ovary) cells were treated with a green fluorescent protein (GFP) alone or a tannylated fluorescent protein prepared by mixing GFP and tannic acid.

As shown in Figure 1, when CHO (Chinese Hamster Ovary) cells were treated with only the fluorescent protein, it was confirmed that the fluorescent protein did not enter the cells at all and was distributed outside the cell membrane (top of Figure 1). On the other hand, when the tannylated fluorescent protein prepared by mixing GFP and tannic acid was treated, it was confirmed that the fluorescent protein was introduced into the cells and bound to the cell nuclei (bottom of Figure 1).

From the above results, it was confirmed that that the tannic acid bound to the protein and thus could easily deliver the protein into cells.

### Experimental Example 2: Evaluation of whether tannic acid can significantly increase activity of botulinum toxin (BTX) itself (confirmation of improving efficacy of Botox)

The electrophysiological measurement (patch-clamp CMAP) was performed as follows to evaluate whether the activity of the botulinum toxin itself was increased when botulinum toxin was tannylated by mixing botulinum toxin (BTX) and tannic acid.

The electrophysiological measurement method and results are as follows.

### (1) Culture of rat cortical neurons

A. SD rats were euthanized with CO₂ gas on the 18^{th} day of pregnancy, and then the cerebral regions were separated from the brain of the 18-day-old embryo from the placenta.
B. The separated brain-cerebral tissues were reacted in 5 mL of digestion solution, specifically, EBSS (Earle's Balanced Salt Solution) containing papain and Dnase I at 37°C for 35 minutes.
C. To the supernatant of the reacted digested cell solution, 5 mL of a blocking solution (FBS + Ovomucoid protease inhibitor + Dnase I + EBSS) was added, followed by centrifugation at 300 g for 3 minutes.
D. After discarding the supernatant, the cell pellet was suspended in 5 mL of the blocking solution, followed by centrifugation at 300 g for 3 minutes.
E. After discarding the supernatant, the cell pellet was suspended in 5 mL of a seeding solution (FBS + B-27 + Glutamax + penicillin/streptomycin + Neurobasal media).
F. The solution was seeded on a 12 mm coverslip coated with poly-D-lysine at the density of 2.0X10⁵ cells/100 *µ*ℓ.
   (seeded on a Axion MEA plate coated with Poly-D-lysine at the density of 1.5X10⁵ cells/100 *µ*ℓ)
G. After 2 hours, 500 *µ*ℓ of the seeding solution was added.
H. The solution was replaced with a feeding solution (B-27 + Glutamax + Neurobasal media) every 2 to 3 days.

### (2) Treatment conditions

A. 100 *µ*ℓ of PBS solution in which 50 units of botulinum neurotoxin type A (BoNT/A, Botulax of Hugel, Inc.) were dissolved was prepared.
B. Tannic acid (TA) was prepared by dissolving it in PBS at a concentration of 25 µM.
C. 50 *µ*ℓ of BoNT/A and 5 *µ*ℓ of TA were mixed, followed by reaction at room temperature for 5 minutes.
D. The reacted BoNT/A and TA solution was treated to make the final culture solution 500 *µ*ℓ.

### (3) Recording of mIPSC (mini inhibitory post synaptic current) using electrophysiological measurement (patch-clamp CMAP)

Glass pipette solution conditions (mM): 135 CsCl, 10 HEPES, 1 EGTA, 1 Na-GTP, 4 and 10 QX-314 (pH 7.4, adjusted with CsOH). The resistance of the tip of a glass pipette was adjusted to be about 4 to 5 Ω. The composition of the solution (mM) that stores neurons when performing electrophysiological recording was as follows: 140 NaCl, 5 KCl, 2 CaCl, 1 MgCl₂, 10 HEPES, 10 glucose (pH 7.4, adjusted with NaOH). To record spontaneous inhibition, CNQX and APV, the spontaneous AMPA and NMDA receptor inhibitors, were added to the solution that stores neurons. Electrical recording was performed using Axon Patch-Clamp (Molecular Devices) devices, and mIPSC recording was performed by fixing the membrane voltage of neurons at -70mV. Analysis was performed using Clampfit 10 (Molecular Devices) and Origin8 software (Mocrocal Inc). Statistical analysis was performed by Student's t-test (*P□<□0.01).

### (4) Axion MEA measurement method

Axion MEA measurement was performed as follows. Before the neuron culture solution was treated with botulinum toxin (BTX), burst frequency and network burst frequency were recorded. 24 hours after the treatment with the botulinum toxin, burst frequency and network burst frequency were recorded again. The results before and after the treatment were compared.

### (5) Experimental results

Experimental results are shown in Figures 2 to 4

Figure 2 is a diagram showing the results of patch-clamp of the group treated with botulinum toxin (BTX) alone (corresponding to BoNT 50U in the diagram) and the group treated with botulinum toxin (BTX) tannylated by treating tannic acid (TA) (corresponding to BoNT 50U / TA 250 nM in the diagram).

Figure 3 is a set of graphs showing the results of deriving LogIC₅₀ values for each treatment concentration of botulinum toxin from the results of patch-clamp of the group treated with botulinum toxin (BTX) alone (corresponding to BoNT Treatment in the diagram) and the group treated with botulinum toxin (BTX) tannylated by treating tannic acid (TA) (corresponding to BoNT/TA Treatment in the diagram).

As shown in Figures 2 and 3, it was confirmed that the effect of the botulinum toxin itself was about 1.8 times higher in the experimental group treated with botulinum toxin (BTX) tannylated by treating tannic acid (TA) than in the experimental group treated with botulinum toxin (BTX) alone. The smaller the LogIC₅₀ value was, the greater the effect of the toxin was. In Figure 3, 0.5, 1.0 and 1.5 of the x-axis mean the botulinum toxin concentration (U/ml).

Figure 4 is a set of graphs showing the results of recording burst frequencies and network burst frequencies of the group treated with tannic acid (TA) alone (corresponding to TA[250] in the diagram), the group treated with botulinum toxin (BTX) alone (corresponding to B[5], B[10] and B[25] in the diagram) and the group treated with botulinum toxin (BTX) tannylated by treating tannic acid (TA) (corresponding to B-T[5], B-T[10] and B-T[25] in the diagram) from Axion MEA data. In the case of TA, the concentration unit is nM, in the case of B, the concentration unit is U (unit), the value in [] of B-T is the concentration of B, and the TA concentration was fixed at 250 nM.

As shown in Figure 4, it was confirmed that the incidence of burst frequency and network burst frequency, the results of recording using Axion MEA, was significantly reduced in the group treated with the tannylated botulinum toxin (BTX) than in the group treated with tannic acid or botulinum toxin (BTX) alone.

For reference, in Figure 4, B[5] means that the concentration of the botulinum toxin was 5 U/ml, B[10] means that the concentration of the botulinum toxin was 10 U/ml, and B [25] means that the concentration of the botulinum toxin was 25 U/ml.

From the electrophysiological results, it was confirmed that the secretion of neurotransmitters (i.e., acetylcholine) was significantly reduced by the botulinum toxin (BTX) in the experimental group treated with the tannylated botulinum toxin, indicating that the activity of the botulinum toxin itself was significantly increased by tannic acid.

### Experimental Example 3: Evaluation of side effect reduction effect of intracutaneous injection of tannylated botulinum toxin (confirmation of reducing effect on Botox side effects)

In order to evaluate whether not only the activity of the botulinum toxin itself is increased but also the side effects that occurred when the botulinum toxin was injected intradermally can be reduced when the botulinum toxin is tannylated by mixing botulinum toxin (BTX) and tannic acid, the following experiment was performed.

The specific experimental method and results are as follows.

### (1) Culture of rat cortical neurons

The equipments used, treatment concentrations, stimulation conditions, and mouse data are as follows, and the experimental process is shown in Figure 5. Figure 5 is a diagram showing the experimental process of an *in vivo* experiment evaluating whether the side effects caused by intradermally injecting botulinum toxin can be reduced when botulinum toxin is tannylated by mixing botulinum toxin (BTX) and tannic acid.
A. Equipments: PowerLab 26T, needle electrode, LabChart software
B. After preparing BoNT/A [100 unit/mL] and TA [100 nM], they were mixed in a volume ratio of 1:1 and reacted (final conc. of BoNT/A: [50 unit/mL], and final conc. of TA: [50 nM]).
C. ICR mice (7 weeks old, 35±3 g) were anesthetized with breathing, and electrical stimulation corresponding to 70V / 4mA / 0.1ms was applied to 2 cm and 5 cm from the tail root. The reaction was measured 10 times by inserting electrodes on the upper and lower ends of the gastrocnemius of the left/right hind legs. At this time, a ground reference electrode was placed at the tail root.
D. After the measurement, 10 *µ*ℓ of BoNT/A or BoNT/A + TA mixture was injected into the gastrocnemius.

### (2) Experimental results

Experimental results are shown in Figure 6.

Figure 6 is a set of graphs showing the results of comparing the possibility of causing side effects by expanding botulinum toxin through blood vessels from the injection site by measuring CMAP electrical waveforms at the injection site and the non-injection site (Vehicle) in the group injected with botulinum toxin (BTX) alone (corresponding to BoNT/A only in the diagram) and the group injected with a mixture of botulinum toxin and tannic acid (corresponding to BoNT/A + TA in the diagram).

As shown in Figure 6, the CMAP electrical waveforms at both the injection site and the non-injection site (Vehicle) were decreased in the group injected with the botulinum neurotoxin alone (left graph). From the above results, it was confirmed that the botulinum neurotoxin could spread through blood vessels from the injection site, and the botulinum neurotoxin could cause side effects even in unintended places.

On the other hand, the CMAP electrical waveforms at the injection site were decreased but the CMAP electrical waveforms at the non-injection site (Vehicle) did not decrease in the group injected with a mixture (composition) containing botulinum neurotoxin and tannic acid (right graph). From the above results, it was confirmed that the tannylated botulinum neurotoxin stayed well in the injected place and it was prevented from spreading to the blood due to the properties of tannic acid, which has high protein-binding ability, so that the side effects of the conventional Botox caused by the spread of the botulinum neurotoxin could be minimized.

The Manufacturing Examples of the composition of the present invention are described hereinafter.

### Manufacturing Example 1: Preparation of pharmaceutical formulations

### <1-1> Preparation of injectable solution

| | |
|---|---|
| Tannylated botulinum toxin complex | 10 mg |
| Mannitol | 180 mg |
| Sterilized distilled water | 2974 mg |
| Na₂HPO₄ · 2H₂O | 26 mg |

Injectable solutions were prepared by mixing all the above components, putting the mixture into 2 mℓ ampoules by the conventional method for preparing injectable solutions.

### <1-2> Preparation of liquid formulations

| | |
|---|---|
| Tannylated botulinum toxin complex | 10 mg |
| Isomerized sugar | 10 g |
| Mannitol | 5 g |
| Purified water | proper amount |

All the above components were dissolved in purified water. After adding lemon flavor, total volume was adjusted to be 100 mℓ by adding purified water. Liquid formulations were prepared by putting the mixture into brown bottles and sterilizing thereof by the conventional method for preparing liquid formulations.

### INDUSTRIAL APPLICABILITY

The botulinum toxin pharmaceutical composition comprising tannic acid provided in one aspect of the present invention improves the activity of the botulinum neurotoxin itself, allows the botulinum neurotoxin to stay in the injected place, and prevents the botulinum neurotoxin from spreading to the blood due to the properties of tannic acid. Therefore, the composition of the present invention has an effect of minimizing the side effects of the conventional Botox caused by the spread of the botulinum neurotoxin.

## Claims

1. A tannylated botulinum toxin complex comprising botulinum toxin (Botox) and tannic acid (TA).

2. The botulinum toxin complex according to claim 1, wherein the botulinum toxin is tannylated by binding to tannic acid.

3. A pharmaceutical composition comprising botulinum toxin (Botox); and tannic acid (TA) as active ingredients.

4. The pharmaceutical composition according to claim 3, wherein the botulinum toxin is tannylated by binding to tannic acid.

5. A pharmaceutical composition comprising botulinum toxin (Botox); and tannic acid (TA) as active ingredients, for use in preventing or treating neuromuscular related diseases.

6. The pharmaceutical composition for use according to claim 5, wherein the neuromuscular related disease is one or more diseases selected from the group consisting of myoclonus, hemifacial spasm, spasmodic torticollis, anal fissure, blepharospasm, facial spasm, cerebral palsy, medication overuse headache, chronic migraine, myalgia, strabismus, temporomandibular disorder, neuralgia, overactive bladder, urge urinary incontinence, rhinitis, acne, dystonia, hyperhidrosis and vocal fold granuloma.

7. A cosmetic composition comprising botulinum toxin (Botox); and tannic acid (TA) as active ingredients.

8. The cosmetic composition according to claim 7, wherein the cosmetic effect of the cosmetic composition is selected from the group consisting of reduced appearance of fine lines, reduced appearance of wrinkles, widening of the eyes, lifting the corner of the mouth, decrease in muscle mass and flattening of the lines extending from the upper lip.

9. A method for treating cosmetic defect comprising a step of administering a cosmetically effective amount of a tannylated botulinum toxin complex comprising botulinum toxin (Botox) and tannic acid (TA) to a subject in need.

10. A use of the tannylated botulinum toxin complex comprising botulinum toxin (Botox) and tannic acid (TA) to prepare a cosmetic composition for the treatment of cosmetic defect.

11. A botulinum toxin (Botox) adjuvant comprising tannic acid (TA) as an active ingredient.

12. A botulinum toxin adjuvant comprising tannic acid (TA) as an active ingredient, for use in the prevention or treatment of one or more diseases selected from the group consisting of myoclonus, hemifacial spasm, spasmodic torticollis, anal fissure, blepharospasm, facial spasm, cerebral palsy, medication overuse headache, chronic migraine, myalgia, strabismus, temporomandibular disorder, neuralgia, overactive bladder, urge urinary incontinence, rhinitis, acne, dystonia, hyperhidrosis and vocal fold granuloma.

13. The botulinum toxin adjuvant according to claim 11, wherein the botulinum toxin adjuvant is used for cosmetic purposes.
